# EUROPEAN PATENT APPLICATION

(11) **EP 1 990 426 A1**
(43) Date of publication of application: **12.11.2008**
(21) Application number: 07710830.6
(22) Date of filing: 24.01.2007
(51) Int. Cl.: C12Q 1/68, C07K 14/435, C12N 15/12

(54) **A METHOD FOR PREPARING THE PRIMERS FOR IN VITRO IDENTIFYING MUTATIONS WITHIN LARGED VESTIBULAR AQUEDUCT SYNDROMIC HEARING LOSS PDS GENE AND THE USE THEREOF**

(30) Priority: 28.02.2006 CN 200610058282
(71) Applicant: Jin, Zhengce, Beijing 100027 (CN)
(72) Inventor: DAI, Pu, Beijing 100027 (CN); HAN, Dongyi, Dongcheng District, Beijing 100027 (CN)
(74) Representative: Ziebig, Marlene
(86) International application number: PCT/CN2007/000260
(87) International publication number: WO 2007/098671

(57) **Abstract**

The present invention relates to a method of preparing a primer used for determining the IVS7-2A→G site mutation involved in the large vestibular aqueduct syndrome. The method comprises the steps of : designing a primer pair which may introduce any base substitutive mutation in the region of 1-13 bases between upstream and downstream including the IVS7-2 mutation site (A→G) based upon the IVS7-2 A→ G mutation site in the PDS gene, so as to obtain a new restriction site which may be used for distinguishing IVS7-2 wild type A site and mutant G site in the amplification products. The present invention further relates to the primers prepared according to this method, the kit or reagent or identical product comprising the primers, and a method for determining the IVS7-2A→G site mutation of large vestibular aqueduct syndrome with said primers in vitro.

Sequence list

<110>   Jin zheng-ce

<120>   A method for preparing primer used for determining the mutation in the PDS gene of
large vestibular aqueduct syndromic hearing loss in vitro and the use of said primer

<160>   4

<170>   PatentIn Version 2.1

<210>   1
<211>   336
<212>   DNA
<213>   Homo Sapiens

<220>
<221>   prim_transcript
<222>   (1)...(336)
<223>   exon7+intron7+exon8 of the PDS gene， the A at position 252 is PDS IVS7-2 site

<400>   1
ACGCTGGTTG   AGATTTTTCA   AAATATTGGT   GATACCAATC   TTGCTGATTT
CACTGCTGGA   60
TTGCTCACCA   TTGTCGTCTG   TATGGCAGTT   AAGGAATTAA   ATGATCGGTT
TAGACACAAA   120
ATCCCAGTCC   CTATTCCTAT   AGAAGTAATT   GTGGTAAGTA   GAATATGTAG
TTAGAAAGTT   180
CAGCATTATT   TGGTTGACAA   ACAAGGAATT   ATTAAAACCA   ATGGAGTTTT
TAACATCTTT   240
TGTTTTATTT   CAGACGATAA   TTGCTACTGC   CATTTCATAT   GGAGCCAACC
TGGAAAAAAA   300
TTACAATGCT GGCATTGTTA AATCCATCCC AAGGGG   336

<210>   2
<211>   4
<212>   DNA
<213>   artificial sequence

<220>
<223>   the restriction recognizing site of Hpa II

<400>   2
CCGG   4

<210> 3
<211> 30
<212> DNA
<213> artificial sequence

<220>
<223> forward primer F1

<400> 3
TGGAGTTTTT AACATCTTTT GTTTTATTCC 30

<210> 4
<211> 20
<212> DNA
<213> artificial sequence

<220>
<223> reverse primer R1

<400> 4
CCCTTGGGAT GGATTTAACA 20

## Description

### Technical field

The present invention relates to a method for preparing primer used for determining PDS gene mutation of large vestibular aqueduct syndrome in vitro. Particularly, the present invention provides a method for preparing primer used for determining the IVS7-2A→G mutation within PDS gene of large vestibular aqueduct syndrome. The present invention further provides a primer prepared according to the method and the kit comprising the primer, and the use of the primer or the kit for determining the IVS7-2A→G mutation within the PDS gene of large vestibular aqueduct syndrome in vitro.

### Background

The incidence of congenital hearing loss of newborn is 1-3/1000. There are many factors leading to hearing loss. More than half of children patients with hearing loss are diagnosed hereditary hearing loss (Wang,Jinling , "Large Vestibular Aqueduct Syndrome and Fluctuating Heating Loss ", Journal of Audiology and Speech Pathology, 2003, 11(2): 81-84).The large vestibular aqueduct syndrome caused by PDS gene (Pendred's syndrome gene) mutation is an important type. In 1978, Valvassori and Clemis found 50 vestibular aqueduct cases by conducting tomography scan in 3700 temporal bones continuously, and named them as large vestibular aqueduct. The clinical symptoms associated with sensorineural hearing loss and large vestibular aqueduct were named as "larged vestibular aqueduct syndrome" (LVAS). The patients had normal hearing when they were born, or accompanied with light to medium hearing loss. The children suffering from the disease had excellent linguistic capacity at the early stage. Then they lost their hearing gradually. Most of the precipitating factors of hearing loss are head trauma, intracranial hypertension, and catch cold, etc. Since falling from bed, infant games or lightly collision in physical exercises can bring about apparent hearing loss, the diagnosis of the disease at the early stage will prevent occurrence and deterioration of it efficaciously, which enable the disease preventable (Wang,Jibao, "Large Vestibular Aquduct Syndrome", Chinese Journal of Otorhinolaryngology Head and Neck Surgery, 2002, 37: 398-400). Recently, many foreign researchers have found that there are close correlations between the large vestibular aqueduct syndrome and the PDS gene mutation (Campbell C, Cucci RA, Green GE, et al. Pendred Syndrome, DFNB4 and PDS----identification for eight novel mutations and phenotype-genotype correlations. Human mutation, 2001,17:403-411; Scott DA, Wang R, Kreman, et al. Functional differences of the PDS gene product are associated with phenotypic variation in patients with Pendred syndrome and non-syndromic hearing loss (DFNB4). (Human Molecular Genetics, 2000, 9:1709-1715). PDS gene (SLC26A4) (Solute carrier family) is composed of 21 exons.The open reading frame of PDS is 2343bp in length, encoding a protein "Pendrin" which consists of 780 amino acids. Pendrin mainly consists of hydrophobic amino acids, belonging to ionic transport family.Studies suggested that the main functions of it is the transportation of iodine/chloride ions. Almost all of the hereditary patterns of PDS gene mutations which lead to hearing loss belong to autosomal recessive heredity. That is to say, parents are carriers, and 1/4 of the offsprings have the disease because they inherit two mutated PDS gene mutations from their parents (homozygote), 2/4 of the offsprings are carriers because they inherit only one mutated PDS gene mutation from their father or mother (heterozygote without disease), and the other 1/4 of the offsprings are wild type individuals for PDS gene because they inherit the two normal PDS alleles from their parents.

We have found 26 patients carrying different PDS mutations with enlarged vestibular aqueduct among 29 Chinese patients under our investigation, 19 patients of which carrying IVS7-2 A→G mutation, indicating that the IVS7-2 A→G mutation is a important causation leading to large vestibular aqueduct syndromee. We have found five IVS7-2 A→G homozygous mutants, four IVS7-2 A→G heterozygous mutants among the PDS gene detection of 63 deaf-mute pupils from Chifeng city of Mongolia, China, the muatation rate is 14.28%. We have found one heterozygous mutant for IVS7-2 A→G among 100 Chinese normal hearing individuals, the estimated carrier rate of this mutation in ordinary Chinese population is about 1%.

We have demonstrated for the first time that the IVS7-2 A→G mutation is the most hot mutation spot of the Chinese patients of large vestibular aqueduct, and the incidence of this disease is at a high level. But no suitable restriction sites of IVS7-2 site in the native sequences of PDS gene were found for identify IVS7-2 A→G mutation and wild type sequences. Conventional method could identify the mutations occurred in this region through sequence analysis only. Although sequence analysis is a standardized method for gene diagnosis, the method is not suitable for screening and popularization because it needs expensive equipment, complex operation, highly skilled persons. It is a time and labor consuming method.

Therefore, a method suitable for wide screening and rapid diagnosis of the IVS7-2 A→G mutation in the PDS gene among hearing loss population is needed.

### Summary of the invention

The present invention was carried out based upon the following principles: distinct primers was designed by using the mutated site (i.e. IVS7-2 A→G at position 252 of SEQ ID NO:1) within the mutated PDS gene related to large vestibular aqueduct syndrome, with or without bio-software. We introduced a new restriction site including the IVS7-2 site in the PDS gene through PCR method artificially, and obtained the accurate results of the IVS7-2 A→G mutation in the PDS gene of large vestibular aqueduct syndrome through analyzing the restriction results of PCR products being tested.

Therefore, according to one aspect of the present invention, a method for preparing primers suitable for determining the IVS7-2A→G site mutation of large vestibular aqueduct syndrome in vitro was provided. I t is composed of primer used for amplifying the IVS7-2 site of PDS gene and with or without bio-software based upon the IVS7-2 A→G mutation site of PDS gene (the inframe of intron 7 is the PDS IVS7-2 site which is the position 252 in SEQ ID NO:1, the PDS IVS 7-2 A-G mutation is the result of the base A which was substituted by base G at this site, the mutation is a splicing site mutation, which will result in the loss of exon 8 during transcription). The primer may introduce a base substitutive mutation in the region of 1-13 bases between upstream and downstream including the A→G mutation at IVS7-2 site, so as to obtain a new restriction site that may be used for distinguishing IVS7-2 wild type (A site) and mutant(G).

As the well-known commercial shortest restriction endonuclease recognition site including 4 bases (e.g. CviJI, recognition site RGCY), the longest restriction endonuclease recognition site including 13 to 14 bases (e.g. Sfi I including 13 bases, StySQI including 14 bases), the primers which may introduce a base substitutive the mutation in the region of 1 to 13 bases between upstream and downstream including the A→G mutation at IVS7-2 site are all useful for the present invention. Accordingly, the length of said region of 1-13 bases between upstream and downstream is preferably 1 to 10 bases, more preferably 1 to 8 bases, even more preferably 2 to 6 bases, most preferably 2-4 bases.

In another aspect, the present invention provides a method used for designing the primer through computer aided primer design software program. The software program used in the invention including, but not limited to Genetool Lite program (a software available from Biotools company, USA), Vector NTI, Oligo, Genestar, DNAMAN, DNASTAR, DNAtool, proteintool, Enzyme, pDRAW, Primer3, Primer5, Seq Convertor, and DNAassist, etc.

In another aspect, the present invention provides primers prepared according to the method mentioned above, including forward primers (F1) and reverse primers (R1). Wherein, the length of the said primers are in the range of 10 to 40bp, preferably 15 to 35bp, more preferably 18 to 32bp.

In another aspect, the present invention provides a particular primer sequence prepared according to the method mentioned above.The Said primer sequence including: the forward primer sequence of 30bp in length (SEQ ID NO:3), 5'TGGAGTTTTTAACATCTTTTGTTTTATTCC 3', wherein, introducing a T-C artificial mutation at the second position of 3' end; and the reverse primer sequence of 20bp in length (SEQ ID NO:4), 5'CCCTTGGGATGGATTTAACA 3'.

In another aspect, the present invention provides kit or identical product which comprising said primers, and can be used for determining the PDS mutated gene of large vestibular aqueduct syndrome in vitro, comprising: PCR amplification reactants,mixture of said forward primer and reverse primer mixed at a ratio of 1:1, novel restriction endonuclease and it's corresponding buffer,positive specimen control, negative specimen control, and operation instruction. Wherein, the primary function of said kit or identical product is that each reagent may be contained in a small box, so that the amplification of nucleic acid fragments, the restriction and identification could be accomplished with the reagents provided in the said kit or identical product sequentially.

The said kit or identical product further comprises kit or reagents used for isolating DNA from blood sample, except for the reagents used for isolating DNA from blood sample, other components in those kit or reagents are identical. Type I kit or reagent comprises solution I of DNA lysis buffer which was used for isolating DNA from plantar blood sample, the principal component is Chelex (5 % Chelex , 0.1%SDS, 1%NP40, 1%Tween 20, product from ABI company); Type II kit or reagent comprising kit (Watson Biotechnologies,Inc)was used for isolating DNA from peripheral blood sample.

In another aspect, the present invention provides a kit used for the newly introduced restriction site HpaII, comprising:
1. Optionally comprising reagent or kit used for isolating DNA from plantar blood or peripheral blood sample;
2. PCR amplification reactants, comprising dNTP, 10XPCR buffer , Mg++, triple distilled water, Taq enzyme;
3. Mixture of the said forward primer (SEQ ID NO:3) and reverse primer (SEQ ID NO:4) mixed at a ratio of 1:1;
4. Restriction enzyme HpaII and its corresponding buffer;
5. Positive specimen control, negative specimen control, and
6. Operation instruction.

In another aspect, the present invention provides a method for determining the IVS7-2 A→G mutation in the PDS gene of large vestibular aqueduct syndrome in vitro, and the method comprises the steps of: Designing primer used for amplify the IVS7-2 site of PDS gene through the method for preparing primers mentioned above, wherein, a base substitutive mutation may be introduced into the said primer in the region of 1 to 13 bases between upstream and downstream including the A→G mutation at IVS7-2 site, so as to obtain a new restriction site which may be used for distinguishing IVS7-2 wild type (A site) and mutant(G); or use the primer prepared through said method directly, or use the said kit/identical product directly, amplify the samples to be detected utilizing said primer, there was a site-directed mutation of a single base between the region near 3' end of the forward primer and amplification template, but it does not affect the efficiency of the PCR of the forward primer and reverse primer, therefore, the amplification products of same length may be obtained with wild type sample or mutant sample; Restriction analysis was carried out on the amplification products with the said novel restriction endonuclease, wherein, the amplification fragments of wild type sample can not be restricted by the said novel restriction endonuclease as it comprises no mutated novel restriction site, and the amplification products of mutant sample can be restricted by the said novel restriction endonuclease as it comprises mutated novel restriction site, so as to and they can identify the sample with IVS7-2 site A-G mutation within the PDS gene.

In the method mentioned above, the length of the said region of 1 to 13 bases between upstream and downstream is preferably 1 to 10 bases, more preferably 1 to 8 bases, even more preferably 2 to 6 bases, most preferably 2 to 4 bases.

In the method mentioned above, it further comprises a step of designing the said primers with computer primer design software program. The software programs,which were used, including, but not limited to: Genetool Liteprogram (a software available from Bio-tools company, USA), Vector NTI, Oligo, Genestar, DNAMAN, DNAtool, Proteintool, Enzyme, pDRAW, Primer3, Primer5, Seq Convertor, and DNAassist, etc.

In another aspect, the present invention provides a method for introducing a new restriction endonuclease HpaII site into the PDS gene, the method comprises the steps of : designing a primer pair used for amplifying IVS7-2 site of the PDS gene (SEQ ID NO:3, 4), wherein, a bases substitutive mutation may be introduced into the region of 2 to 4 bases between upstream and downstream including the IVS7-2 site (A→G), so as to obtain a new restriction site HapII which may be used for distinguishing IVS7-2 wild type (A site) and mutant(G) ; isolating the DNA of the sample to be detected, and obtaining amplification templates as needed; PCR amplification was carried out with amplification templates using the said primer pair (SEQ ID NO:3, 4); amplification fragments of 114bp in length may be produced through amplification with both wild type and PDS IVS7-2 A→G mutatant samples, a bright band of more than 100bp in length was found in the actual PCR products in agarose electrophoresis, which confirmed the mismatch between the second base at 3' end of forward primer (SEQ ID NO:3)3' and the template does not affect the efficiency of PCR; Restricting the PCR products with HpaII, wherein, the PCR products comprising a PDS IVS7-2 site , if the template is a IVS7-2 A→G mutant molecule, then a HpaII restriction site exists in the amplification products of 114bp in length, and two sets of bands of 83 to 85bp and 29 to 31bp in length were obtained after restricted with HpaII. If the template is a PDS IVS7-2 wild type molecule, then no HpaII restriction site exists in the amplification products of 114bp in length, and it can not be restricted by HpaII. The band of 114bp maintains intact after restriction; Determining whether a IVS7-2 A→G mutation in the PDS gene of large vestibular aqueduct syndrome is existing in the sample to be detected according to the results of restriction electrophoresis or not.

In the method for determining new restriction endonuclease HpaII site, wherein the designing of primer pair may be accomplished by computer primer design software, such as Genetool Liteprogram (a software available from Biotools company, USA), Vector NTI, Oligo, Genestar, DNAMAN, DNAtool, Proteintool, Enzyme, pDRAW, Primer3, Primer5, Seq Convertor, and DNAassist, etc.

In the method for determining new restriction endonuclease HpaII site, wherein the extraction, PCR amplification, and restriction of sample DNA may be accomplished with kits or reagents comprising the said primer pair (SEQ ID NO:3 or 4) and new restriction endonuclease HpaII site.

The method for determining PDS gene IVS7-2 A→G mutation of the present invention provides the following advantages comparing with currently available methods (sequencing method) :
The operation steps of the restriction method with restriction endonuclease of the present invention were simple, and can be completed within 4 hours; the reagents involved are common biochemical reagents, and low cost; the PCR apparatus and electrophoresis devices are basic equipment of biology laboratories and examination laboratories. These characteristics are advantage to the screening of PDS gene IVS7-2 A→G mutation nationwide, and can prevent large vestibular aqueduct syndrome, thereby reducing the possibility of deafness of large vestibular aqueduct syndromic individual fundamentally. The sequence analysis method needs sequencer, and the technical operation is complex. It takes 3 to 7 days to finish one complete analysis.

### Description of the figures

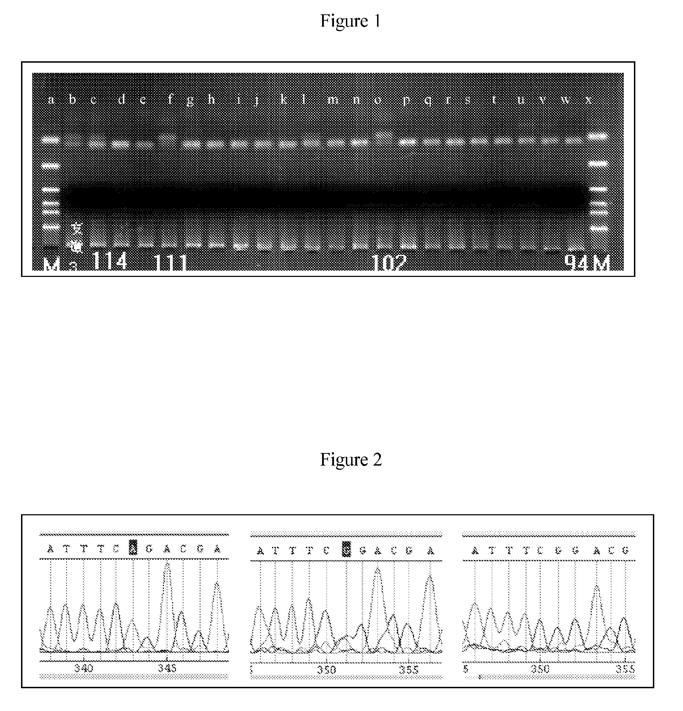

Figure 1 dispicts the electrophoretic pattern for identifying the IVS7-2 A→G mutation of HpaII which restricted PDS gene on 2.5% agarose gel, lane b, c: individual's heterozygous for the IVS7-2 A→G mutation in the PDS gene; lane f, o: individual's homozygous for the IVS7-2 A→G mutation in the PDS gene; lane a, x: markers (interval was 100bp); the rest lanes : normal wild type individuals.
Figure 2 dispicts the results of sequence analysis which confirmed the accuracy of restriction results. Left : keep intact after restriction, no IVS7-2A-G mutation appeared in sequencing (eg. lane d); middle: two bands appeared after restriction, sequence analysis confirmed that it was heterozygous for the IVS7-2A-G mutation (lane b, c); right: all of the bands were restricted after restriction or the brightness of smaller bands were stronger than that bigger ones, sequence analysis confirmed that it was homozygous for the IVS7-2A-G mutation (lane f, o).

### Detailed Description

Now, the present invention will be described in more detail in referred to the following nonlimited embodiments. It should be understood that, the following embodiments are merely exemplary, and is in no way intended to limit the present invention. Unless otherwise stated, the embodiments of the present invention utilizing conventional molecular biology, cell biology, PCR amplification and mutagenesis technologies, etc., which is well known to the skilled in the art, and described in a number of literatures in detail. See, for example, Sambrook and Russell" Molecular Cloning: A Laboratory Manual" (2001); Cloning: A Practical Approach, " Volumes I and II(D.N.Glover, ed., 1985)"; T.A Brown "Genome" BIOS Scientific Publishers Limited.

### Example 1 The determination of the IVS7-2 mutation in the PDS gene of deaf-mute population

### 1.Examination of specimen

66 deaf-mutes(all from School for deaf-mutes of Chifeng, China), 100 individuals with normal hearing were selected, with DNA isolated from peripheral whole blood of all subjects by the method provided in the kit(Watson Biotechnologies,Inc), Then the specimens are examined. The deaf-mutes were confirmed to be severe or profound hearing loss by pure-tone audiometry.

### 2.Primer design

The guiding ideology of primer design is introducing a base substitution in the region of 2 to 4 bases around the PDS IVS7-2 site (i.e. Position 252 in SEQ ID NO:1), so as to obtain a new restriction site which may be used for distinguishing IVS7-2 wild type (A) and mutant(G). The GenetoolLite program was used in primer design , the length of forward primer is 30bp, a T-C mutation was introduced into the PDS IVS7-4 site, the length of reverse primer is 20bp, complementing with the wild type PDS gene forward sequence completely. The forward primer sequence (SEQ ID NO:3) is 5'TGGAGTTTTTAACATCTTTTGTTTTATTCC 3', The reverse primer sequence (SEQ ID NO:4) is 5'CCCTTGGGATGGATTTAACA 3'. The amplification fragment of 114bp can be produced by amplification both with wild type specimen and PDS IVS7-2 A→G mutation specimen. The PCR products comprise the PDS IVS7-2 site. If the template is a IVS7-2 A→G mutant molecule, there is a HpaII restriction site in the amplification products of 114bp in length, and two sets of bands of 83 to 85bp and 29 to 31bp were obtained after HpaII restriction; If the template is a PDS IVS7-2 wild type molecule, there is no HpaII restriction site in the amplification products of 114bp in length, and it can not be restricted by HpaII, the band of 114bp maintaining intact after restriction.

### 3.PCR amplification reaction

Forward primers F and reverse primer R were obtained through artificial synthesis(synthesized with automatic nucleic acid synthesizer using given sequences) based upon said designed forward and reverse primer sequences, and said DNA isolated from detected peripheral blood was used as templates for PCR amplification reactions:

### Reaction system:

| | |
|---|---|
| Templates | 50ng |
| Primers F1, R1 | each 50ng |
| 10×dNTP | 5µl |
| 10×Buffer | 5µl |
| Final concentration of Mg⁺⁺ | 1.5mmol/L |
| Taq enzyme | 1.0u |
| Add triple distilled water to | 50µl. |

### Reaction conditions:

Denaturating for 5 minutes at 95°C, then denaturating for 30 seconds at 94°C, annealing 30 seconds at 55°C, extension 30 seconds at 72°C, 30 circles together, extension 7 minutes at 72°C finally.

The products were analyzed through 2.5% agarose gel electrophoresis. A bright and defined band bigger than the band of 100bp was obtained after PCR amplification with F1R1, and indicate that the mismatch between the second base at 3' end of the forward primer and the templates does not affect the efficiency of PCR.

### 4. HpaII restriction analysis

The PCR products comprise the PDS gene IVS7-2 site. If the template is a IVS7-2 A→G mutant molecule, there is a HpaII restriction site in the amplification products of 114bp in length, and two sets of bands of 83 to 85bp and 29 to 31bp were obtained after HpaII restriction(only one band of 83 to 85bp appearing on the gel); If the template is a mt IVS7-2 wild type molecule, there is no HpaII restriction site in the amplification products of 114bp in length, and it can not be restricted by HpaII, the band of 114bp maintains intact after restriction.

Analyzing the IVS7-2A→G mutation of the PCR amplification products from specimen is detected under the following conditions.

### Restriction reaction system:

| | |
|---|---|
| PCR products | 12-18 µl |
| 10×Buffer | 3 µl |
| HpaII restriction enzyme | 5-10u |
| BSA | 0.3 µl |

Adding triple distilled water to 30µl, incubated for one hour at 37°C. Analyzing the restricted reactants with 2.5% agarose gel.

### 5.Results

During the gel electrophoretic analysis after restriction, only one band bigger than 100bp was found in 57 cases among the selected 66 deaf-mutes, indicated that no IVS7-2A→G mutation existed; a band bigger than 100bp and a band smaller than 100bp were found in 4 cases, the brightness of the bigger band was stronger than the smaller one, indicated that they are heterozygous for IVS7-2 A→G mutation; a band smaller than 100bp was found in the rest 5 cases, indicated that they are homozygous for IVS7-2 A→G mutation (see Figure 1 for some data);

### 6. The assay for reliability of HpaII restriction method

Analyzing the sequences of exon 7 and 8 of the restricted DNA samples from 5 cases which were confirmed comprising homozygous mutation for IVS 7-2 A→G of PDS gene directly, indicated that homozygous A→G substitution was existed for IVS 7-2 site; Analyzing the sequences of exon 7 and 8 of the restricted DNA samples from 4 cases which were confirmed comprising heterozygous mutation for IVS 7-2 A →G of PDS gene directly, indicated that heterozygous A→G substitution was existed for IVS 7-2 site; Analyzing the sequences of exon 7 and 8 of the restricted DNA samples from 11 cases which were confirmed comprising no mutation for IVS 7-2 A →G of PDS gene directly, indicated that the IVS 7-2 site was normal (Figure 2). Dilated vestibular aqueduct were found in the 9 cases of IVS 7-2 A→G mutation individuals (homozygous or heterozygous) through temporal bone CT scan analysis, no abnormity was found in the negative individual (table 1).

**Table 1. Comparison for the results of IVS 7-2A→G kit detection, sequence analysis and temporal bone CT scan analysis**

| mutant type | Method provided in the IVS 7-2A→ G kit | sequence analysis | temporal bone CT |
|---|---|---|---|
| homozygous | 5 cases | 5 cases | 5 cases with dilated vestibular aqueduct |
| heterozygous | 4 cases | 4 cases | 4 cases dilated vestibular aqueduct |
| negative | 11 cases | 11 cases | 11 cases with no abnormity |

### Example 2 The determination of the IVS7-2 mutation in the PDS gene of the DNA extracted from dried plantar blood spot collected on filter paper

### 1.A type of kit useful for dried plantar blood spot collected on filter paper (suitable for 100 persons) comprises the following components:

(1) Solution I (the principal component is 5% Chelex) 25ml;
(2)PCR reagents (comprising 8mM dNTP 150µl, 10×PCR buffer 1ml, 25mM Mg⁺⁺ solution 1ml, triple distilled water 1ml×4 tubes, 5u/µl Taq enzyme , 25µl);
(3)Forward primers F1; Reverse primers R1(the same as in example 1), forward and reverse primers mixed at a ratio of 1:1 (concentration was 10nM for each), total 150µl;
(4)Restriction enzyme HpaII, 20u/µl, 110µl and the corresponding 10× buffer, 1ml;
(5)Positive control specimen 125µl, negative control specimen 125µl;
(6)Operation instruction.

### 2. Subjects analyzed

One patient who has been confirmed to be large vestibular aqueduct syndrome by CT examination and carried the PDS gene homozygous IVS7-2 A→G mutation, the parents with normal hearing (they are carriers of heterozygous mutation), and two individuals with normal hearing and without IVS7-2 A→G mutation in the PDS gene were chosen for analysis.

### 3. Analyzing methods and results

According to the operation instruction provided in the kits, isolating DNA from dried plantar blood spot collected on filter paper of said subjects with solution I (The principal component is 5% Chelex), and taking 50ng DNA used as templates, adding100ng of primers mixture, 10×dNTP 5µl, 10× buffer 5µl, the final concentration of Mg⁺⁺ is 1.5mmol/L, adding triple distilled water to 50µl, then 1.0u Taq enzyme was added, Denaturating for 5 minutes at 95°C, then denaturating for 30 seconds at 94°C, annealing 30 seconds at 55°C, extending 30 seconds at 72°C, 30 circles together, extending 7 minutes at 72°C finally. PCR reaction were carried out under the same PCR reaction conditions, both the positive template and negative template of PDS gene with IVS7-2 A→G heterozygous mutation provided in the kit were used as positive and negative controls, and PCR blank control tubes without template were provided, analyzing the amplification products through 2.5% agarose gel electrophoresis after reaction, as a result, no amplification products were found in the control without templates, the patient, his/her parents, individuals with normal hearing, the PCR products of positive and negative control specimen are 114bp in length. Analyzing the PCR products restricted with HpaII, the volume of restriction reaction system is 30µl, and comprising:

| | |
|---|---|
| 10× buffer | 3µl |
| 100 BSA | 0.3µl |
| HpaII | 20u |
| PCR products | 12-18µl |

Made up the volume with triple distilled water, incubated at 37°C for 1 hour. Analyzing the restricted reactants through 2.5% agarose gel electrophoresis, a band of 83-85bp was found in the restricted products from said one patient with large vestibular aqueduct syndrome, indicated that homozygous IVS7-2 A→G mutation was existed in the PDS gene; a band of 83-85bp and a band of 114bp were found both in the restricted products from the parents of the patient with large vestibular aqueduct syndrome and the restricted products from the positive control, indicated that heterozygous IVS7-2 A→G mutation existed in the PDS gene; only one 114bp band was found in said two individuals with normal hearing and the negative control, indicated that no IVS7-2 A→G mutation existed in the PDS gene. Sequence analysis showed that the patient is homozygous for the IVS7-2 A→G mutation, and the parents are heterozygous for the IVS7-2 A→G mutation representing their carrier condition. Said two individuals with normal hearing do not carry such IVS7-2 A→G mutations.

### Example 3 Two type of kits for determining IVS7-2 mutation in the PDS gene in vitro and the use of it

The subjects chosen for analyzing were identical to that of example 2. Except that the specimen tested was peripheral blood, the extractant used for isolating blood DNA is commercialized kit used for isolating DNA from peripheral blood, and use the kit in isolating DNA from peripheral blood, the rest components comprised in the kit and the analyzing method were the same as in example 2. The results were the same as in example 2. That is, a band of 83-85bp was found in the restricted products from said one patient with large vestibular aqueduct syndrome, indicated that homozygous IVS7-2 A→G mutation was existed in the PDS gene; a band of 83-85bp and a band of 114bp were found both in the restricted products from the parents of the patient with large vestibular aqueduct syndrome and the restricted products from the positive control, indicated that heterozygous IVS7-2 A→G mutation existed in the PDS gene; only one 114bp band was found in said two individuals with normal hearing and the negative control, indicated that no IVS7-2 A→G mutation existed in the PDS gene.

### Example 4 Three types of kits for determining IVS7-2 mutation in the PDS gene in vitro and the use of it

The subjects chosen for analyzing were identical to that of example 2. Except that the specimen tested was DNA which was isolated from peripheral blood, and the kit do not comprise the extractants used for isolating blood DNA. The rest components comprised in the kit and the analyzing method were the same as in example 2. The results were the same as in example 2 as well.

### Industrial applicability

The present invention is composed of primers or kits used for determining the PDS gene mutation of large vestibular aqueduct syndrome in vitro, which may be used in the rapid screen, the rapid diagnosis and the prevention of large vestibular aqueduct syndrome caused by the IVS7-2 A→G mutation could reduce the possibility of hearing loss occurred in the large vestibular aqueduct syndrome individuals.

## Claims

1. A method of preparing a primer pair used for determining the IVS7-2A→G site mutation of the large vestibular aqueduct syndrome in vitro, **characterized in that** the method comprising the steps of : designing the forward primer and reverse primer used for amplificating SEQ ID NO:1, which comprising the IVS7-2 mutation sites A →G at position 252 based upon the sequences of the seventh and the eighth exons, and the sequence of seventh intron between the above exons of the PDS gene as set forth in SEQ ID NO:1, with or without bio-software, wherein the primer pair may introduce a base substitutive mutation in the region of 1-13 bases between upstream and downstream of said mutated site, so as to obtain a new restriction site which may be used for distinguishing IVS7-2 wild type A site and mutant G site in the amplification products .

2. The method according to claim 1, wherein a T-C artificial mutation was introduced into the second position of the 3' end of the forward primer in respect of the PDS gene amplification templates, so as to obtain a new restriction site HpaII as set forth in SEQ ID NO:2 in the amplification products.

3. A primer pair prepared according to the method of claim 1 or 2, wherein the length of forward primer or reverse primer of said primer pair is 10-40bp, preferably 15 to 35bp, more preferably 18 to 32bp.

4. The primer pair according to claim 3, wherein forward primer possessing the sequence as set forth in SEQ ID NO:3 and a T-C artificial mutation was introduced into the second position of the 3' end, reverse primer possessing the sequence as set forth in SEQ ID NO:4, a new restriction endonuclease HpaII site may be introduced into said primer pair at the IVS7-2 mutated site A→G of the PDS gene.

5. The use of the primer pair according to claim 3 or 4 in the preparation of a kit or identical product for determining the IVS7-2A→G site mutation comprised in the large vestibular aqueduct syndrome in vitro.

6. A kit /identical product comprising the primer pair according to claim 3 or 4 which was used for determining the IVS7-2A→G site mutation comprised in the large vestibular aqueduct syndrome in vitro, said kit /identical product comprising:
(1)PCR amplification reactants;
(2)Mixture of the forward primer and reverse primer according to claim 3 or 4 which mixed at a ratio of 1:1;
(3)Novel restriction endonuclease and its corresponding buffer;
(4) Optionally positive specimen control, negative specimen control; further comprising
(5) Operation instruction.

7. The kit /identical product according to claim 6, comprises a reagent or kit used for isolating DNA from blood sample.

8. The kit /identical product according to claim 7, wherein the reagent or kit used for isolating DNA from blood sample comprises type I kit or reagent used for isolating DNA from plantar blood sample, comprising Solution I of DNA lysis buffer , the principal component of it is Chelex; or type II kit or reagent comprises the reagent used for isolating DNA from peripheral blood sample.

9. A method for determining IVS7-2 A→G mutation in the PDS gene of large vestibular aqueduct syndrome in vitro, comprises:
(1) Preparing a primer pair through the method according to any one of claims 1-2, and used in the PCR amplification of the sample, or using the primer pair according to any one of claim 3 and claim 4 in the amplification of the sample directly, or amplifying the wild type sample and mutant sample with the kit /identical product according to any one of claim 6 to claim 8 directly, to obtain amplification products of the same length;
(2) Restricting the amplification products with said restriction endonuclease, based upon the new restriction endonuclease site introduced into the amplification products by the primer pair;
(3) Analyzing the restriction products through electrophoresis, a number of bands were obtained from the amplification products which comprising IVS7-2 A→G mutation after restriction as a result of new restriction endonuclease site comprised in it, and only one single band was obtained from the amplification products without IVS7-2 A→G mutation after restriction as a result of no new restriction endonuclease site comprised in it;
(4) Determining whether a IVS7-2 A→G mutation in the PDS gene of large vestibular aqueduct syndrome exist in the sample to be detected or not, based upon the results of restriction electrophoresis.

10. The method according to claim 9, wherein said primer pair are forward primer as set forth in SEQ ID NO:3 and reverse primer as set in SEQ ID NO:4; said novel restriction enzyme is HapII; said amplification products with the same length are the amplification products of 114bp in length; two sets of bands of 83 to 85bp and 29 to 31bp were obtained from the amplification products comprising the IVS7-2 A→G mutation after restriction, and only one band of 114bp was obtained from the amplification products without the IVS7-2 A→G mutation after restriction.
